# EUROPEAN PATENT APPLICATION

(11) **EP 4 137 168 A1**
(43) Date of publication of application: **22.02.2023**
(21) Application number: 21825810.1
(22) Date of filing: 15.06.2021
(51) Int. Cl.: A61L 27/36, A61L 27/54, A61L 27/56, A61L 33/00, A61L 33/04

(54) **VALVE MATERIAL HAVING LONG-ACTING ANTITHROMBOSIS PROPERTY AND PREPARATION METHOD THEREFOR**

(30) Priority: 15.06.2020 CN 202010542260
(71) Applicant: Venus MedTech (HangZhou), Inc., Hangzhou, Zhejiang 310052 (CN)
(72) Inventor: WANG, Yunbing, Hangzhou, Zhejiang 310052 (CN); LUO, Rifang, Hangzhou, Zhejiang 310052 (CN); YANG, Li, Hangzhou, Zhejiang 310052 (CN); ZHANG, Fanjun, Hangzhou, Zhejiang 310052 (CN)
(74) Representative: KIPA AB
(86) International application number: PCT/CN2021/100182
(87) International publication number: WO 2021/254347

(57) **Abstract**

The present invention provides a valve material having a long-acting antithrombosis property and a preparation method therefor. The preparation method therefor comprises the following steps: performing glutaraldehyde cross-linking treatment on an animal-derived biological valve material, so that the valve material can resist decomposition for a long time; soaking the treated valve material in a formulation solution containing a cross-linking agent and a modifier for 10-60 min, then increasing the temperature to 30-60°C, and performing heat treatment for 1-12 h; and rinsing the valve material after heat treatment, so as to obtain the valve material. The valve material prepared by the method has excellent antithrombosis and anti-calcification properties, and can effectively solve the problem of calcification and thrombosis in the valve material treated by existing means of glutaraldehyde cross-linking. The valve material prepared by the present method can be used as a valve material required for aortic valve, pulmonary valve, venous valve, mitral valve and tricuspid valve replacement.

## Description

### TECHNICAL FIELD

The invention belongs to the technical field of modifications to biomedicine materials and medical devices, in particular to valve materials with long-acting antithrombosis properties and preparation methods thereof. The valve materials prepared according to the present methods can be used as valve materials for aortic valve, pulmonary valve, venous valve, mitral valve and tricuspid valve replacement.

### BACKGROUND

Heart valve disease (HVD) refers to a disease with abnormal heart function, which is caused by the diseased native valve that affects the normal blood flow. With the development of economy and the aging of population, the incidence of heart valve disease presents a rising trend. According to an uncompleted statistic, in China, the number of patients with aortic valve diseases annually increased by about 200,000. The incidence of heart valve disease in the elderly is 13.3%, just lower than the hypertension and coronary heart diseases. Valve replacement is the final solution to valve diseases, especially for severe patients. Transcatheter valve therapy is becoming the mainstream in valve replacement, and the relative technologies include aortic valve, pulmonary valve, mitral valve, tricuspid valve and venous valve replacements. Currently, the valve materials used in interventional procedures are mainly xenograft valve materials, which are heart valves and pericardium derived from animal (mainly porcines and bovines) and crosslinked with cross-linking agents such as glutaraldehyde.

Valve replacement requires high performances of valve materials, and the requirements are different depending on different valve practices. However, mechanical properties, anti-calcification performance, fatigue life and so on are the most essential requirements. In particular, the anti-clotting is particularly required by pulmonary valve and venous valve materials as pulmonary valve and venous valves involve complex hemodynamic environment, wherein the circulating blood is venous blood with a slow flow rate and easy to clot. Unfortunately, despite the disadvantages of glutaraldehyde cross-linked valves, such as valvular thrombus and calcification, the commercialized valve materials are still mainly cross-linked by glutaraldehyde, due to the detoxification, sterilization and preservation performances of the glutaraldehyde cross-linked valve. Thus, there is a need for pulmonary artery / venous valve materials with an improved blood compatibility and thus with an excellent long-acting antithrombosis property in clinical practice, which are based on the antithrombosis modification technology through glutaraldehyde crosslinking, with great value in clinical practice and market.

### SUMMARY

In view of the above problems in the prior art, the present invention provides a valve material having a long-acting antithrombosis property and a preparation method thereof, which can effectively solve the problems that the valve materials prepared by the existing glutaraldehyde cross-linking methods are easy to calcification and clot. The valve materials prepared according to the present invention can be used as valve materials for replacement of aortic valve, pulmonary valve, venous valve, mitral valve and tricuspid valve.

For the above objectives, the present invention provides the following technical solution to solve the above technical problems:

A method for preparing a valve material having a long-acting antithrombosis property includes the steps of:
(1) Crosslinking an animal-derived biological valve material with glutaraldehyde;
(2) Immersing the valve material treated in step (1) into a formulated solution containing a cross-linking agent and a modifier, and performing heat treatment; and
(3) Rinsing the valve material treated in step (2), thereby obtaining the valve material having a long-acting antithrombosis property.

A method for preparing a valve material having a long-acting antithrombosis property includes the steps of:
(1) Crosslinking the animal-derived biological valve material with glutaraldehyde so that the valve material can resist decomposition for a long time;
(2) Immersing the valve material treated in step (1) into a formulated solution containing a cross-linking agent and a modifier for 10-60 min, and then heating to 30-60 °C for heat treatment for 1-12 h; and
(3) Rinsing the valve material treated in step (2), thereby obtaining the valve material having a long-acting antithrombosis property.

Further, the animal-derived biological valve material includes one of porcine pericardium, bovine pericardium and small intestinal submucosa.

Further, the concentration of glutaraldehyde in step (1) is in the range of 0.3-3%, the crosslinking time is in the range of 24-96 h, and the crosslinking pH value is in the range of 6-9.

Further, the concentration of glutaraldehyde in step (1) is 1%, the crosslinking time is 72 h, and the crosslinking pH value is 7.

Further, the crosslinking method in step (1) includes one-step crosslinking, multi-step crosslinking and concentration gradient crosslinking, and the crosslinking solvent is water, PBS or other salt ion buffers.

Further, in step (2), the valve material treated in step (1) is immersed in a formulated solution containing a cross-linking agent and a modifier for 30 minutes, and then heated to 40 °C for heat treatment for 6 hours;

Further, the solvent in the formulated solution in the step (2) is one or more selected from a group consisting of hexafluoroisopropanol, isopropanol, ethanol, methanol and water.

Further, the cross-linking agent is one or more selected from a group consisting of 1-ethyl-3-(3-dimethylaminopropyl) carbodiimide hydrochloride, N-hydroxysuccinimide, metaphosphate, isocyanate and maleimide hydrazide.

Further, the cross-linking agent in step (2) includes one or more of 1-ethyl-3-(3-dimethylaminopropyl) carbodiimide hydrochloride, metaphosphate, isocyanate and maleimide hydrazide.

Further, the cross-linking agent is one or more of 1-ethyl-3-(3-dimethylaminopropyl) carbodiimide hydrochloride and maleimide hydrazide.

Further, the modifier in step (2) is a small molecule of phospholipid compound or a polymer containing phospholipid groups.

Further, in step (2), the phospholipid compound is glycerophosphate choline, lecithin, dimyristoyl phosphatidylcholine, 1,2-diacyl-sn-glycero-3-phosphocholine, 2- (methacryloyloxy) ethyl-2- (trimethylamino) ethyl phosphate, or methylacryloyloxyethyl sulfobetaine.

Further, the polymer containing phospholipid groups in step (2) is lauryl methacrylate, butyl methacrylate, N- (3-aminopropyl) methacrylamide or methacrylamide.

Further, the modifier is a phosphocholine compound or a copolymer of a phosphocholine compound and an auxiliary monomer compound.

Further, the phosphocholine compound is at least one of glycerophosphate choline, lecithin, dimyristoyl phosphatidylcholine, 1,2-diacyl-sn-glycero-3-phosphocholine, 2-(methacryloyloxy) ethyl-2- (trimethylamino) ethyl phosphate, and methylacryloyloxyethyl sulfobetaine.

Further, the auxiliary monomer compound is at least one of lauryl methacrylate, butyl methacrylate, N- (3-aminopropyl) methacrylamide and methacrylamide.

Further, the cross-linking agent is sodium hexametaphosphate and the modifier is glycerophosphate choline.

Further, the cross-linking agent is isocyanate, the modifier is a copolymer of methylacryloyloxyethyl sulfobetaine, lauryl methacrylate and butyl methacrylate.

Further, the cross-linking agent is sodium metaphosphate, and the modifier is a copolymer of lauryl methacrylate and 2- (methacryloyloxy) ethyl-2- (trimethylamino) ethyl phosphate.

Further, the cross-linking agent is 1-ethyl-3-(3-dimethylaminopropyl) carbodiimide hydrochloride, and the modifier is a copolymer of 2- (methacryloyloxy) ethyl-2-(trimethylamino) ethyl phosphate and N-(3-aminopropyl) methacrylamide.

Further, the cross-linking agent is a mixture solution of 1-ethyl-3-(3-dimethylaminopropyl) carbodiimide hydrochloride, N-hydroxysuccinimide and sodium metaphosphate, and the modifier is a mixture solution of lecithin and dimyristoyl phosphatidylcholine.

Further, the mass concentration of the cross-linking agent in the formulated solution of step (2) is in the range of 0.05 wt% to 5 wt%.

Further, the mass concentration of the modifier in the formulated solution in step (2) is in the range of 0.2 wt% to 2 wt%.

Further, the mass concentrations of the cross-linking agent and the modifier in the formulated solution in step (2) are in the range of 0.05 wt% to 5 wt% and in the range of 0.2 wt% to 2 wt%, respectively.

Further, the mass concentrations of the cross-linking agent and the modifier in the formulated solution in step (2) are in the range of 0.05 wt% to 2 wt% and in the range of 0.2 wt% to 1 wt%, respectively.

Further, the molar ratio of the cross-linking agent to the modifier of the formulated solution in step (2) is in the range of 0.5-2 : 0.2-1.

Further, the molar ratio of the cross-linking agent to the modifier of the formulated solution in step (2) is 1 : 1.

Further, in step (2), the treated valve material is immersed in the formulated solution containing the cross-linking agent and the modifier for 10-60 min and then the heat treatment is performed.

Further, in step (2), the treated valve material is immersed in the formulated solution containing the cross-linking agent and the modifier, and then is heated to 30-60 °C for heat treatment for 1-12 h.

Further, in step (2), the treated valve material is immersed in the formulated solution containing the cross-linking agent and the modifier for 10-60 min, and then heated to 30-60 °C for heat treatment for 1-12 h.

A valve material with a long-acting antithrombosis property is prepared according to the above method.

The present invention has the following advantages:
The biological valve material is derived from pericardium tissue of animal origin with fiber orientation and porous structure, with groups such as carboxyl groups, amine groups and hydroxyl groups. By cross-linking with glutaraldehyde, the amine groups on the valve material are greatly consumed so that aldehyde groups may be remained on the valve material, which causes a potential risk of calcium ion attachment and the further formation of calcium salt crystals, leading to calcification. In the present invention, by introducing the cross-linking agent on the valve material, the residual aldehyde groups are directly blocked by a schiff base reaction between the amino groups of the cross-linking agent and the aldehyde groups, avoiding calcium ions residue and improving the anti-calcification ability of the valve material; the residual aldehyde groups and hydroxyl groups on the valve material can also be combined with the phosphocholine component by covalent cross-linking, thereby introducing the component with anti-clotting property; the phosphocholine component involves anti-clotting groups of the biomimetic membrane with a phospholipid bilayer, which can further improve the anti-clotting performance of the valve material.

### BRIEF DESCRIPTION OF THE DRAWINGS

FIG. 1 shows thrombosis on the surface of glutaraldehyde cross-linked valve material;
FIG. 2 shows thrombosis on the surface of modified valve material; and
FIG. 3 (left figure) shows calcification of glutaraldehyde cross-linked valve material after subcutaneous implantation for 3 months, and FIG. 3 (right figure) shows calcification of modified valve material after subcutaneous implantation for 3 months.

### DESCRIPTION OF THE EMBODIMENTS

The specific embodiments of the present invention will be described in detail with reference to the drawings.

### EXAMPLE 1

A valve material having a long-acting antithrombosis property is prepared through the following steps:
(1) Cut a defatted porcine pericardium material into an appropriate size, stretch the material and then immerse it into a glutaraldehyde solution with a concentration of 1%, renew the solution after 24 hours, and repeat the treatment for 72 hours;
(2) Wash the obtained cross-linked valve material, and immerse the valve material into a formulated solution for 30 minutes, wherein the solvent of the formulated solution is hexafluoroisopropanol, the cross-linking agent is sodium hexametaphosphate, and the modifier is glycerophosphate choline. The concentration of the sodium hexametaphosphate is 1 wt%, and the concentration of the glycerophosphate choline is 0.5 wt%; then the valve immersed in the formulated solution is heated to 40 °C for 4 h; and
(3) Wash the valve material obtained in step (2) sufficiently with deionize water to obtain the desired valve material, and preserve the material in a glutaraldehyde solvent for use.

### EXAMPLE 2

A valve material having a long-acting antithrombosis property is prepared through the following steps:
(1) Cut a defatted porcine pericardium material into an appropriate size, stretch the material and then immerse it into a glutaraldehyde solution with a concentration of 0.5% for 24 hours, and then transfer the material into a glutaraldehyde solution with a concentration of 1% for 48 hours;
(2) Wash the obtained cross-linked valve material, and immerse the valve material into a formulated solution for 60 minutes, wherein the solvent of the formulated solution is isopropanol, the cross-linking agent is isocyanate, and the modifier is a copolymer of methylacryloyloxyethyl sulfobetaine, lauryl methacrylate and butyl methacrylate. The concentrations of the isocyanate and the methylacryloyloxyethyl sulfobetaine, lauryl methacrylate and butyl methacrylate in the copolymer are 0.1 wt%, 0.1 wt%, 0.2 wt% and 0.2 wt%, respectively; and then the valve immersed with the formulated solution is heated to 60 °C for 3 h; and
(3) Wash the valve material obtained in step (2) sufficiently with deionize water to obtain the desired valve material, and preserve the material in a glutaraldehyde solvent for use.

### EXAMPLE 3

A valve material having a long-acting antithrombosis property is prepared through the following steps:
(1) Cut a defatted bovine pericardium material into an appropriate size, stretch the material and then immerse it into a glutaraldehyde solution with a concentration of 1% for 24 hours, and then transfer the material into a glutaraldehyde solution with a concentration of 0.5% for 48 hours;
(2) Wash the obtained cross-linked valve material, and immerse the valve material into a formulated solution for 20 minutes, wherein the of the formulated solution is water, and the cross-linking agent is sodium metaphosphate, and the modifier is a copolymer of lauryl methacrylate and 2- (methacryloyloxy) ethyl-2- (trimethylamino) ethyl phosphate. The concentrations of sodium metaphosphate, and the lauryl methacrylate and 2- (methacryloyloxy) ethyl-2- (trimethylamino) ethyl phosphate in the copolymers are 1 wt%, 0.2 wt% and 0.3 wt%, respectively; and then the valve immersed with the formulated solution is heated to 50 °C for 8 h; and
(3) Wash the valve material obtained in step (2) sufficiently with deionize water to obtain the desired valve material, and preserve the material in a mixed solvent of isopropanol and glycerol for use.

### EXAMPLE 4

A valve material having a long-acting antithrombosis property is prepared through the following steps:
(1) Peel off and cut a small intestinal submucosa into an appropriate size, stretch the material and then immerse it into a glutaraldehyde solution with a concentration of 1% for 72 hours;
(2) Wash the obtained cross-linked valve material, and immerse the valve material into a formulated solution for 40 min, wherein the solvent of the formulated solution is isopropanol, the cross-linking agent is 1-ethyl-3-(3-dimethylaminopropyl) carbodiimide hydrochloride, and the modifier is a copolymer of 2- (methacryloyloxy) ethyl-2- (trimethylamino) ethyl phosphate and N-(3-aminopropyl) methacrylamide. The concentrations of 1-ethyl-3-(3-dimethylaminopropyl) carbodiimide hydrochloride and the 2- (methacryloyloxy) ethyl-2- (trimethylamino) ethyl phosphate and N-(3-aminopropyl) methacrylamide in the copolymer are 0.05 wt%, 0.2 wt% and 0.5 wt%, respectively; and then the valve immersed with the formulated solution is heated to 60 °C for 8 h; and
(3) Wash the valve material obtained in step (2) sufficiently with deionize water to obtain the desired valve material, and preserve the material in a glutaraldehyde solvent for use.

### EXAMPLE 5

A valve material having a long-acting antithrombosis property is prepared through the following steps:
(1) Cut a defatted porcine pericardium material into an appropriate size, stretch the material and then immerse it into a glutaraldehyde solution with a concentration of 1% for 72 hours;
(2) Wash the obtained cross-linked valve material, and immerse the valve material into a formulated solution for 10 minutes, wherein the solvent of the formulated solution is water, the cross-linking agent is a mixture solution of 1-ethyl-3-(3-dimethylaminopropyl) carbodiimide hydrochloride / N-hydroxysuccinimide and sodium metaphosphate with concentrations of 0.1 wt% and 2 wt%, respectively, and the modifier is a mixture solution of lecithin and dimyristoyl phosphatidylcholine with concentrations of 0.3 wt% and 0.4 wt%, respectively; and then the valve immersed with the formulated solution is heated to 40 °C for 12 h; and
(3) Wash the valve material obtained in step (2) sufficiently with deionize water to obtain the desired valve material, and preserve the material in a mixed solvent of isopropanol and glycerol for use.

### TEST EXAMPLE

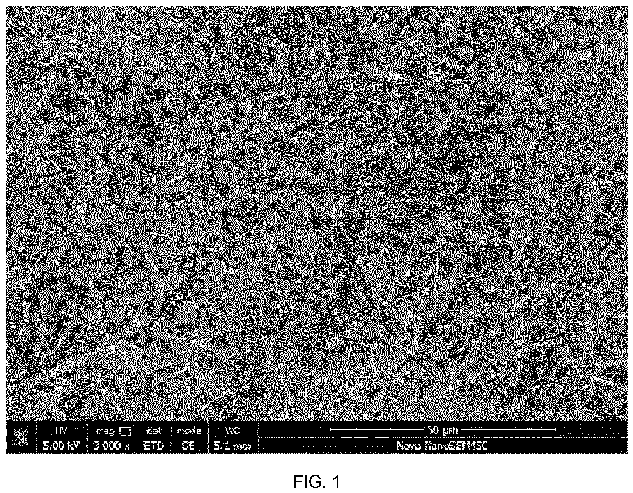

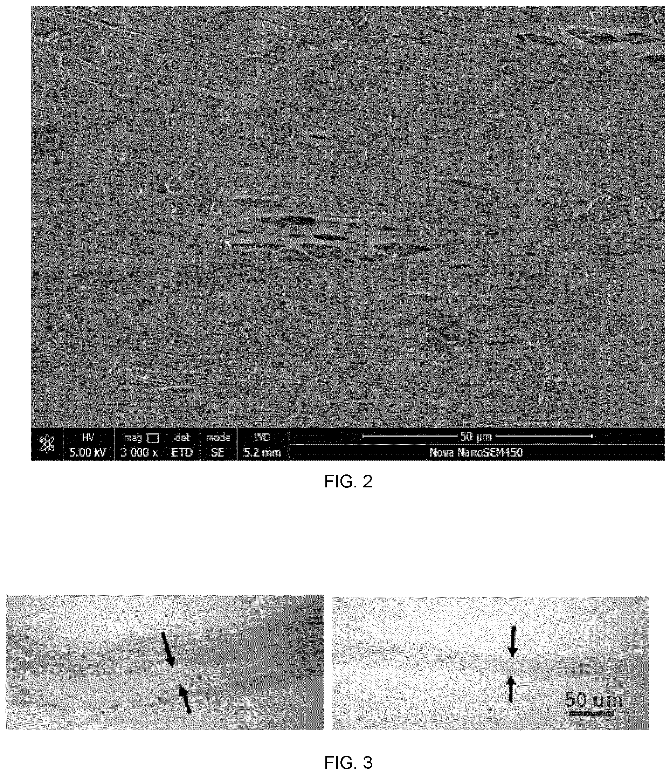

Taking the valve material prepared in EXAMPLE 4 as an example, the structure on the surface of the glutaraldehyde cross-linked valve material is observed, as shown in FIG. 1; then the valve material is treated by modifier and cross-linking agent, the treated valve material suffers from a blood circulation test of rabbit ex vivo for 2 hours, and then thrombosis on the surface of the valve material is observed, as shown in FIG. 2.

As can be seen from FIG. 1, the conventional glutaraldehyde cross-linked valve material without modification is deposited with a large amount of thrombus, red blood cells and a large amount of criss-crossed fibrous. However, FIG. 2 shows that after the anti-clotting modification described in EXAMPLE 4, the surface of the valve material is still smooth without significant thrombosis, proving the excellent blood compatibility of the modified valve material.

As shown in FIG. 3, after subcutaneous implantation for 3 months and staining with alizarin red, there are significant calcified plaques on the glutaraldehyde cross-linked valve material (left figure), while the modified valve material (right figure) is still smooth without significant calcified plaques, which proves the anti-calcification property of the valve material.

## Claims

1. A method for preparing a valve material having a long-acting antithrombosis property, comprising steps of:
(1) crosslinking an animal-derived biological valve material with glutaraldehyde;
(2) immersing the valve material treated in step (1) into a formulated solution containing a cross-linking agent and a modifier, and performing heat treatment; and
(3) rinsing the valve material treated in step (2), thereby obtaining the valve material having a long-acting antithrombosis property.

2. The method for preparing a valve material having a long-acting antithrombosis property of claim 1, wherein the cross-linking agent is one or more selected from a group consisting of 1-ethyl-3-(3-dimethylaminopropyl) carbodiimide hydrochloride, N-hydroxysuccinimide, metaphosphate, isocyanate and maleimide hydrazide.

3. The method for preparing a valve material having a long-acting antithrombosis property of claim 1, wherein the cross-linking agent is one or more selected from a group consisting of 1-ethyl-3-(3-dimethylaminopropyl) carbodiimide hydrochloride and maleimide hydrazide.

4. The method for preparing a valve material having a long-acting antithrombosis property of claim 1, wherein the modifier is a phosphocholine compound or a copolymer of a phosphocholine compound and an auxiliary monomer compound.

5. The method for preparing a valve material having a long-acting antithrombosis property of claim 1, wherein the phosphocholine compound is at least one selected from a group consisting of glycerophosphate choline, lecithin, dimyristoyl phosphatidylcholine, 1,2-diacyl-sn-glycero-3-phosphocholine, 2- (methacryloyloxy) ethyl-2- (trimethylamino) ethyl phosphate, and methylacryloyloxyethyl sulfobetaine.

6. The method for preparing a valve material having a long-acting antithrombosis property of claim 1, wherein the auxiliary monomer compound is one or more selected from a group consisting of lauryl methacrylate, butyl methacrylate, N- (3-aminopropyl) methacrylamide and methacrylamide.

7. The method for preparing a valve material having a long-acting antithrombosis property of claim 1, wherein the cross-linking agent is sodium hexametaphosphate and the modifier is glycerophosphate choline.

8. The method for preparing a valve material having a long-acting antithrombosis property of claim 1, wherein the cross-linking agent is isocyanate, the modifier is a copolymer of methylacryloyloxyethyl sulfobetaine, lauryl methacrylate and butyl methacrylate.

9. The method for preparing a valve material having a long-acting antithrombosis property of claim 1, wherein the cross-linking agent is sodium metaphosphate, and the modifier is a copolymer of lauryl methacrylate and 2- (methacryloyloxy) ethyl-2- (trimethylamino) ethyl phosphate.

10. The method for preparing a valve material having a long-acting antithrombosis property of claim 1, wherein the cross-linking agent is 1-ethyl-3-(3-dimethylaminopropyl) carbodiimide hydrochloride, and the modifier is a copolymer of 2- (methacryloyloxy) ethyl-2-(trimethylamino) ethyl phosphate and N-(3-aminopropyl) methacrylamide.

11. The method for preparing a valve material having a long-acting antithrombosis property of claim 1, wherein the cross-linking agent is a mixture solution of 1-ethyl-3-(3-dimethylaminopropyl) carbodiimide hydrochloride, N-hydroxysuccinimide and sodium metaphosphate, and the modifier is a mixture solution of lecithin and dimyristoyl phosphatidylcholine.

12. The method for preparing a valve material having a long-acting antithrombosis property of claim 1, wherein the cross-linking agent of the formulated solution in step (2) has a mass concentration in a range of 0.05 wt% to 5 wt%.

13. The method for preparing a valve material having a long-acting antithrombosis property of claim 1, wherein the modifier of the formulated solution in step (2) has a mass concentration in a range of 0.2 wt% to 2 wt%.

14. The method for preparing a valve material having a long-acting antithrombosis property of claim 1, wherein a molar ratio of the cross-linking agent to the modifier of the formulated solution in step (2) is 0.5-2 : 0.2-1.

15. The method for preparing a valve material having a long-acting antithrombosis property of claim 1, wherein a solvent of the formulated solution in step (2) is one or more selected from a group consisting of hexafluoroisopropanol, isopropanol, ethanol, methanol and water.

16. The method for preparing a valve material having a long-acting antithrombosis property of claim 1, further comprising, in step (2), immersing the treated valve material in the formulated solution containing the cross-linking agent and the modifier for 10-60 min and then performing the heat treatment.

17. The method for preparing a valve material having a long-acting antithrombosis property of claim 1, further comprising, in step (2), immersing the treated valve material in the formulated solution containing the cross-linking agent and the modifier, and then heating to 30-60 °C for the heat treatment for 1-12 h.

18. The method for preparing a valve material having a long-acting antithrombosis property of claim 1, further comprising, in step (2), immersing the treated valve material in the formulated solution containing the cross-linking agent and the modifier for 10-60 min, and then heating to 30-60 °C for the heat treatment for 1-12 h.

19. The method for preparing a valve material having a long-acting antithrombosis property of claim 1, wherein the animal-derived biological valve material comprises one of porcine pericardium, bovine pericardium and small intestinal submucosa.

20. The method for preparing a valve material having a long-acting antithrombosis property of claim 1, wherein the glutaraldehyde in step (1) has a concentration in a range of 0.3-3%.

21. The method for preparing a valve material having a long-acting antithrombosis property of claim 1, further comprising crosslinking the animal-derived biological valve material with glutaraldehyde for 24-96 h at a pH value in a range of 6-9.

22. A valve material having a long-acting antithrombosis property, which is prepared by the method according to one of claims 1 to 21.
